(19) Europäisches Patentamt / European Patent Office / Office européen des brevets

(11) **EP 4 039 183 B1**

(12) **EUROPEAN PATENT SPECIFICATION**

(45) Date of publication and mention
of the grant of the patent:
**18.10.2023 Bulletin 2023/42**

(21) Application number: **21786072.5**

(22) Date of filing: **26.03.2021**

(51) International Patent Classification (IPC):
*A61B 5/021* (2006.01)    *A61B 5/022* (2006.01)
*A61B 5/024* (2006.01)    *A61B 5/00* (2006.01)

(52) Cooperative Patent Classification (CPC):
**A61B 5/02116; A61B 5/022; A61B 5/02416;**
**A61B 5/681; A61B 5/6826; A61B 5/6843;**
**A61B 5/6898;** A61B 5/021; A61B 5/02405;
A61B 5/02427; A61B 5/0816; A61B 5/1172;
A61B 5/14551; A61B 2562/0247

(86) International application number:
**PCT/CN2021/083437**

(87) International publication number:
**WO 2022/121162 (16.06.2022 Gazette 2022/24)**

(54) **BIOMETRIC INFORMATION DETECTION DEVICE AND ELECTRONIC APPARATUS**

VORRICHTUNG ZUR ERFASSUNG BIOLOGISCHER EIGENSCHAFTEN UND ELEKTRONISCHES GERÄT

DISPOSITIF DE DÉTECTION D'INFORMATIONS BIOMÉTRIQUES ET APPAREIL ÉLECTRONIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.12.2020 PCT/CN2020/135013**

(43) Date of publication of application:
**10.08.2022 Bulletin 2022/32**

(73) Proprietor: **SHENZHEN GOODIX TECHNOLOGY CO., LTD.**
**Shenzhen, Guangdong 518045 (CN)**

(72) Inventor: **HOU, Zhiming**
**Guangdong 518045 (CN)**

(74) Representative: **Herrero & Asociados, S.L.**
**Cedaceros, 1**
**28014 Madrid (ES)**

(56) References cited:
WO-A1-2019/051108     CN-A- 103 228 205
CN-A- 105 686 818     CN-A- 113 080 913
CN-U- 204 520 705     CN-U- 205 697 733
CN-U- 207 855 679     CN-U- 212 539 480
GB-A- 2 578 120     US-A1- 2013 296 714
US-A1- 2014 364 749     US-A1- 2017 360 374

## Description

### TECHNICAL FIELD

An embodiment of the present application relates to the technical field of biological recognition, and more particularly, to a biological characteristic information detection device and an electronic device.

### BACKGROUND

In recent years, with the rapid development of electronic technology and the increasing demand of users for functions of various electronic devices, how to monitor biological characteristic information of a human body through the electronic device in real time so as to make users know their own body conditions at any time and play a role in preventing diseases has attracted widespread attention.

However, some electronic devices, such as smartphones, are becoming thinner, so the internal space of the electronic device is very tight. Therefore, how to accurately detect biological characteristic information in the limited internal space of the electronic device is an urgent problem to be solved.

GB2578120A discloses a clamp comprising first and second clamp portions in which one or both are moveable relative to one another to exert a clamping force on an object. The clamp comprises one or more strain sensors secured to the clamp in a sensing region of the clamp that deforms in response to the clamping force. A signal from strain sensors may be used to detect motion of a clamped object.

US2013/296714A1 discloses a sensing device comprising an electromagnetic wave emitter for emitting electromagnetic waves to a surface; an electromagnetic wave detector for detecting the emitted electromagnetic waves that are reflected from the surface; and a force transmitting member configured to transmit a force applied thereto for detection.

US2014/364749A1 discloses blood pressure measurement devices comprising a transducer may have error introduced when the transducer is placed in mechanical communication with a vein or artery. This error may be based on alignment, applanation, calibration, and the contact based stress required to obtain a signal from pressure in a vein or artery. The present invention teaches isolating and removing this error from the blood pressure system which may increase the accuracy of the measurement. Calibration is also provided.

### SUMMARY

The invention is set out in the appended set of claims.

An embodiment of the present application provides a biological characteristic information detection device and an electronic device. The detection device can accurately detect the biological characteristic information with a small size and is convenient to carry.

### BRIEF DESCRIPTION OF THE DRAWINGS

Embodiments falling under the claimed invention are described in figure 11. The embodiments in figures 9-10 do not fall under the scope of the claimed invention.

FIG. 1 is a schematic diagram of a biological characteristic information detection device according to an embodiment of the present application.
FIG. 2 is an amplitude-time schematic diagram of converting an optical signal reflected by a finger into an electrical signal.
FIG. 3 is a schematic structural diagram of an integrated cantilever beam according to an embodiment of the present application.
FIG. 4 is a schematic structural diagram of a split cantilever beam according to an embodiment of the present application.
FIG. 5 is a schematic diagram of a biological characteristic information detection device according to an embodiment of the present application.
FIG. 6 is another schematic diagram of a biological characteristic information detection device according to an embodiment of the present application.
FIG. 7 is a schematic circuit diagram of a full-bridge circuit formed by strain gauges according to an embodiment of the present application.
FIG. 8 is another schematic three-dimensional diagram of a biological characteristic information detection device according to an embodiment of the present application.
FIG. 9 is another schematic diagram of a biological characteristic information detection device according to an embodiment of the present application.
FIG. 10 is yet another schematic diagram of a biological characteristic information detection device according to an embodiment of the present application.
FIG. 11 is yet another schematic diagram of a biological characteristic information detection device according to an embodiment of the present application.
FIG. 12 is yet another schematic diagram of a biological characteristic information detection device according to an embodiment of the present application.
FIG. 13 is a schematic structural diagram of a biological characteristic information detection device according to an embodiment of the present application applied to a watch.
FIG. 14 is a schematic structural diagram of an electronic device according to an embodiment of the present application.

## DESCRIPTION OF EMBODIMENTS

The technical solutions in embodiments of the present application are described below with reference to the accompanying drawings.

A fingertip blood pressure detection device is an existing device for measuring fingertip blood pressure. The equipment generally requires a spring structure or an airbag structure to apply pressure to the fingertip and then detect the fingertip blood pressure through a sensor. The fingertip blood pressure detection device not only needs an enlarged auxiliary structure and occupies a large space, but also needs to be carried by users daily for use, which brings great inconvenience for the users. In addition, the blood pressure detected by the fingertip blood pressure detection device may be inaccurate.

In view of this, embodiments of the present application provide a biological characteristic information detection device (for convenience of description, hereinafter referred to as a detection device), which can accurately detect the biological characteristic information with a small size and is convenient to carry.

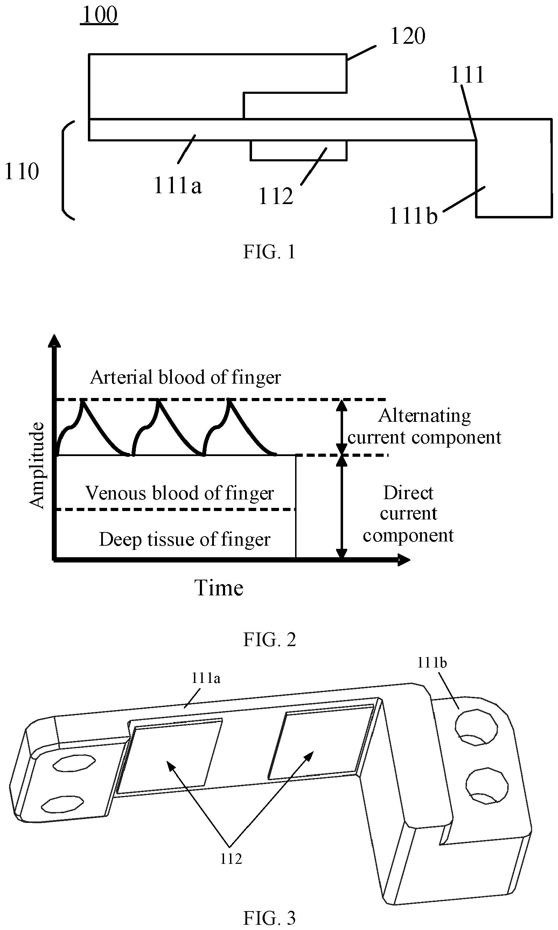

The detection device according to the embodiment of the present application is described in detail below with reference to FIG. 1 to FIG. 13.

It should be noted that for convenience of description, in the embodiments of the present application, the same reference numerals represent the same parts, and for brevity, detailed description of the same part is omitted in different embodiments. It should be understood that thickness, length and other sizes of various parts shown in the accompanying drawings in the embodiments of the present application, and overall thickness, length and other sizes of an integrated device are only exemplary descriptions and should not constitute any limitation to the present application.

FIG. 1 is a schematic diagram of a detection device 100 according to an embodiment of the present application. The detection device 100 may be applied to an electronic device. The detection device 100 may include:

a pressure detection module 110, including a cantilever beam 111 and a pressure sensor 112, where the pressure sensor 112 is fixed on the cantilever beam 111, the cantilever beam 111 includes an elastic arm 111a and a fixed base 111b, the fixed base 111b is fixed in the electronic device, and the elastic arm 111a is suspended in the air;

a photo plethysmography (PPG) detection module 120, arranged at the elastic arm 111a and configured to detect a PPG signal of a user when the user presses electronic device,

where when the user presses the electronic device, a pressure signal applied to the electronic device by the user may be transmitted to the cantilever arm 111 through the PPG detection module 120, so that pressure sensor 112 detects the pressure signal, and the pressure signal and the PPG signal are used to detect biological characteristic information of the user.

The PPG signal is introduced simply below. Light has a certain attenuation after penetrating skin tissues (such as finger skin) and then being reflected and absorption of the light by muscles, bones, veins and other connecting tissues is basically unchanged, but blood is different. Since blood flows in arteries, the absorption of the light by the blood naturally changes. It may be seen from FIG. 2 that the absorption of light by venous blood of the finger or deep tissue of the finger is basically unchanged, so an electrical signal converted by the light detector is a direct current component; however, the absorption of light by arterial blood of the finger is changed, so the electrical signal converted by the light detector is an alternating current component. The alternating current component extracted from the electrical signal converted by the light detector, namely the PPG signal, may reflect the characteristic of blood flow.

Optionally, the biological characteristic information may blood pressure. Of course, the biological characteristic information may be heart rate, blood oxygen, heart rate variability (HRV), respiratory rate and the like.

Optionally, the cantilever beam 111 may be a thin cantilever beam. The thin cantilever beam has a small size, so that a size of the detection device 100 can be further reduced.

Exemplarily, the cantilever beam 111 may have a thickness of 0.1mm-0.6mm. The thickness of the cantilever beam is 0.1mm-0.6mm, so the miniaturization of the detection device 100 is realized, and the detection device 100 may be applied to a miniaturized electronic device, such as a watch and the like.

As shown in FIG. 3, the cantilever beam 111 may be of an integrated structure, that is, the elastic arm 111a and the fixed base 111b are integrally connected.

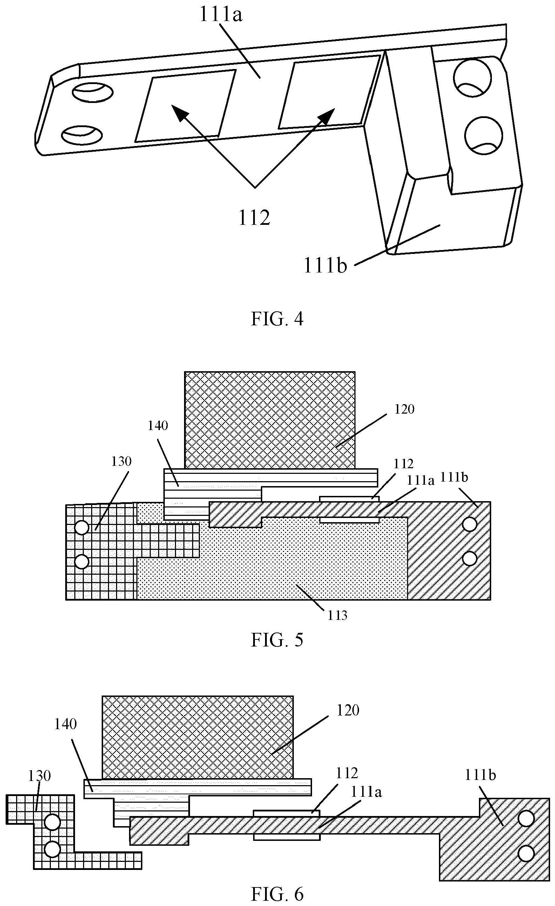

Or as shown in FIG. 4, the cantilever beam 111 may be of a split structure, that is, the elastic arm 111a and the fixed base 111b are two separate parts, and the elastic arm 111a and the fixed base 111b are fixedly connected. Exemplarily, the elastic arm 111a and the fixed base 111b may be integrally fixed through welding, and may also be fixed through an injection molding mode or other modes. The cantilever beam with the split structure is simpler in manufacturing process and easier to perform mass production in industry.

Optionally, referring to FIG. 5, the pressure detection module 110 may further include a module holder 113, and the fixed base 111b of the cantilever beam 111 may be fixed in the electronic device through the module holder 113. That is, the pressure detection module 110 and the PPG detection module 120 may be fixed in the electronic device through the module holder 113. This fixing mode is easy to realize the modular design of the detection device. Other manufacturers may directly use the detection device provided by the embodiment of the present application without making any change to their own products, so that the use scene of the detection device provided by the embodiment of the present application is effectively increased.

As another example, as shown in FIG. 6, the cantilever beam 111 may also be directly fixed in the electronic device, that is, the pressure detection module 110 and the PPG detection module 120 may be directly fixed in the electronic device through the fixed base 111b of the cantilever beam 111. The detection device adopting the fixing mode may be fixed in the electronic device without additionally increasing other parts (such as the module holder shown in FIG. 5), so that the detection device has a simpler structure, lower manufacturing cost and a smaller size.

The fixed base 111b may be provided with a fixing hole. For example, the fixed base 111b in FIG. 6 is provided with two fixing holes, and the fixed base 111b may be fixed in the electronic device through the fixing holes. Of course, the fixed base 111b may be fixed in the electronic device by other modes.

Considering that the user may press the electronic device strongly in some cases, in order to prevent serious deformation of the cantilever beam 111 caused by strong pressing of the user, continuously referring to FIG. 5 and FIG. 6, the detection device 100 provided by the embodiment of the present application may further include a protective stop block 130, where the protective stop block 130 is arranged on one side of the elastic arm 111a away from the fixed base 111b in a first direction and at least partially overlaps the cantilever beam 111 in the first direction; and the first direction is a direction parallel with the elastic arm 111a of the cantilever beam 111.

It may be seen from FIG. 5 that the protective stop block 130 may also be fixed in the electronic device through the module holder 113, or as shown in FIG. 6, the protective stop block may also be directly fixed in the electronic device.

The foregoing content that the protective stop block 130 at least partially overlaps the cantilever beam 111 in the first direction may be understood that: the elastic arm 111a of the cantilever beam 111 at least partially overlaps with the protective stop block 130 in the first direction;

or as shown in FIG. 5 and FIG. 6, the detection device 100 may further include a supporting member 140, the PPG detection module 120 is fixed on an upper surface of the supporting member 140, a part of a lower surface of the supporting member 140 is fixedly connected to the elastic arm 111a, and the remaining part of the lower surface of the supporting member 140 is suspended in the air. The supporting member 140 at least partially overlaps the protective stop block 130 in the first direction.

By setting the supporting member, the PPG detection module can be fixed on the supporting member, so that the stability of the PPG detection module is improved.

It should be noted that in the embodiment of the present application, "upper" refers to one side facing towards the electronic device for sensing the pressing of the user, and "lower" refers to a direction opposite to one side facing towards the electronic device for sensing the pressing of the user.

The embodiment of the present application does not specifically limit the fixing mode between the PPG detection module 120 and the supporting member 140. For example, the PPG detection module 120 may be fixed on the supporting member 140 through a dispensing mode, or the PPG detection module 120 may be fixed on the supporting member 140 through a mechanical mode, such as a screw.

By the protective stop block, the problem that the pressure signal detected by the detection device is invalid due to serious deformation of the cantilever beam caused by the fact that the user presses the electronic device strongly is avoided, so that the accuracy of detecting the biological characteristic information can be further improved.

Optionally, in the embodiment of the present application, the pressure sensor 112 may include, but is not limited to: a piezoelectric pressure sensor, a piezoresistive pressure sensor, a capacitive pressure sensor and an inductive pressure sensor. The embodiment of the present application is described below by taking the case where the pressure sensor 112 is the piezoresistive pressure sensor and specifically includes a strain gauge as an example, and the strain gauge may be a resistance strain gauge, but it should be understood that the embodiment of the present application is not limited to this.

The number of the strain gauge may be at least one, for example, four or eight, and the like. The at least one strain gauge may form a single-arm bridge circuit, a double-arm bridge circuit or a full-bridge circuit. Preferably, the number of the strain gauges is four, and the four strain gauges can form the full-bridge circuit, so that the problem of pressure imbalance may be overcome, and it is ensured that the detection can accurately detect the biological characteristic information of the user no matter the user presses any position in the pressing area of the electronic device. Further, the full-bridge circuit has high sensitivity and can realize temperature self-compensation.

The four strain gauges may be arranged randomly. For example, the four strain gauges may be arranged on an upper surface of the elastic arm 111a; or the four strain gauges may be arranged on a lower surface of the elastic arm 111a; or two strain gauges of the four strain gauges are arranged on the upper surface of the elastic arm 111a and the other two strain gauges are arranged on the lower surface of the elastic arm 111a. Two strain gauges of the four strain gauges are arranged on the upper surface of the elastic arm 111a and the other two strain gauges are arranged on the lower surface of the elastic arm 111a, so that the four strain gauges may easily form the full-bridge circuit, and the manufacturing process of the detection device can be simplified.

The strain gauges may adhere to the elastic arm 111a through a first glue layer. The first glue layer may be a glue layer formed by uniformly coating and solidifying organic glue (such as epoxy glue), and may also be a glue film or organic solid glue with high light transmittance. The glue layer occupies a small space, so that the size of the detection device 100 may be further reduced.

Or the strain gauges may be fixed on the elastic arm 111a through a mechanical mode. For example, the strain gauges may be fixed on the elastic arm 111a through threaded connection.

When the user presses the electronic device, the cantilever beam 111 is stressed, the elastic arm 111a will bend and deform, the strain gauges fixed on the upper surface of the elastic arm 111a are stress and stretched, and the strain gauges fixed on the lower surface of the elastic arm 111a are stressed and compressed, so that the resistance of the strain gauges changes, and the pressure signal applied to the electronic device by the user may be acquired by detecting an output voltage of the full-fridge circuit.

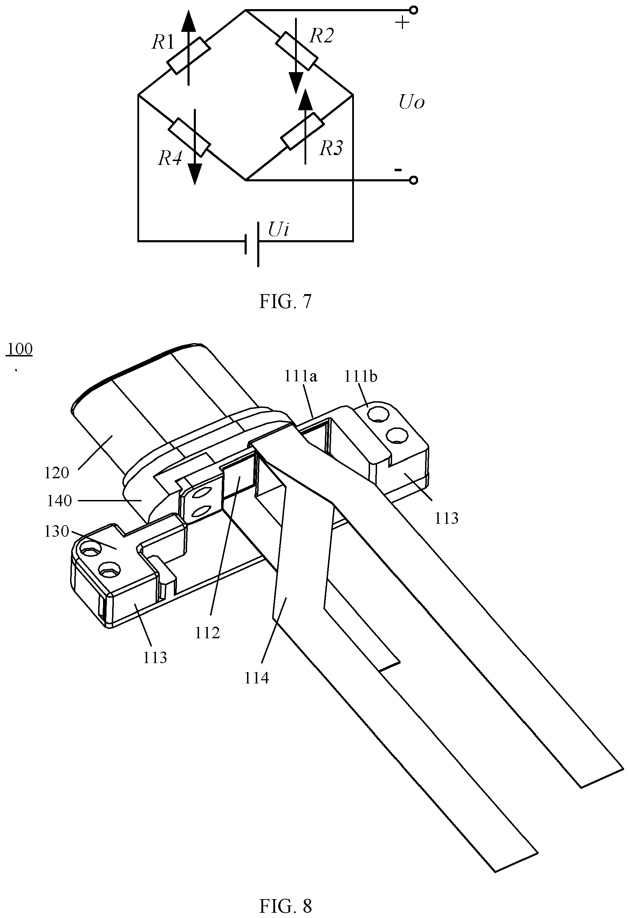

Suppose the four strain gauges are a strain gauge 1, a strain gauge 2, a strain gauge 3 and a strain gauge 4 respectively, the strain gauge 1 and the strain gauge 3 are fixed on the upper surface of the elastic arm 111a, the strain gauge 2 and the strain gauge 4 are fixed on the lower surface of the elastic arm 111a, and the full-bridge circuit formed by the four strain gauges may be shown in FIG. 7. In FIG. 7, the up arrow represents that the strain gauges are stressed and stretched, the down arrow represents that the strain gauges are stressed and compressed, and resistance values of the four strain gauges are R1, R2, R3 and R4 respectively, where R1=R2=R3=R4. The output voltage Uo of the full-bridge circuit meets the following formula:

$$U_o = U_i K \varepsilon$$

where K is a sensitivity coefficient of the strain gauges, $\varepsilon$ is the strain quantity of the strain gauges under the pressure signal applied to the electronic device by the user, and the strain quantity of the strain gauges is equivalent to the resistance change quantity of the strain gauges.

Optionally, as shown in FIG. 8, the pressure detection module 110 may further include a first connecting member 114, where one end of the first connecting member 114 is connected to the cantilever 111 and the other end of the first connecting member 114 is connected to the main board of the electronic device, and the pressure detection module 110 may transmit the electrical signal (for example, Uo in FIG. 7) corresponding to the pressure signal to the main board of the electronic device through the first connecting member 114.

Exemplarily, the first connecting member 114 may be a flexible printed circuit (FPC). The FPC is soft, so a pressure error caused introduced by the FPC is small, the accuracy rate of the pressure signal acquired by the detection device may be ensured, and the accuracy rate of the detected biological characteristic information of the user is further ensured.

More exemplarily, the first connecting member 114 may be a cable. For example, the first connecting member 114 may be a part formed by seven cables.

It should be understood that specific examples in the embodiments of the present application are only intended to help those skilled in the art to better understand the embodiments of the present application and do not limit the scope of the embodiments of the present application.

It should also be understood that in the embodiments of the present application, "first" and "second" are only for distinguishing different objects, but do not limit the scope of the embodiments of the present application.

The pressure detection module 110 of the embodiments of the present application is introduced above, and the PPG detection module 120 of the embodiments of the present application is described in detail below.

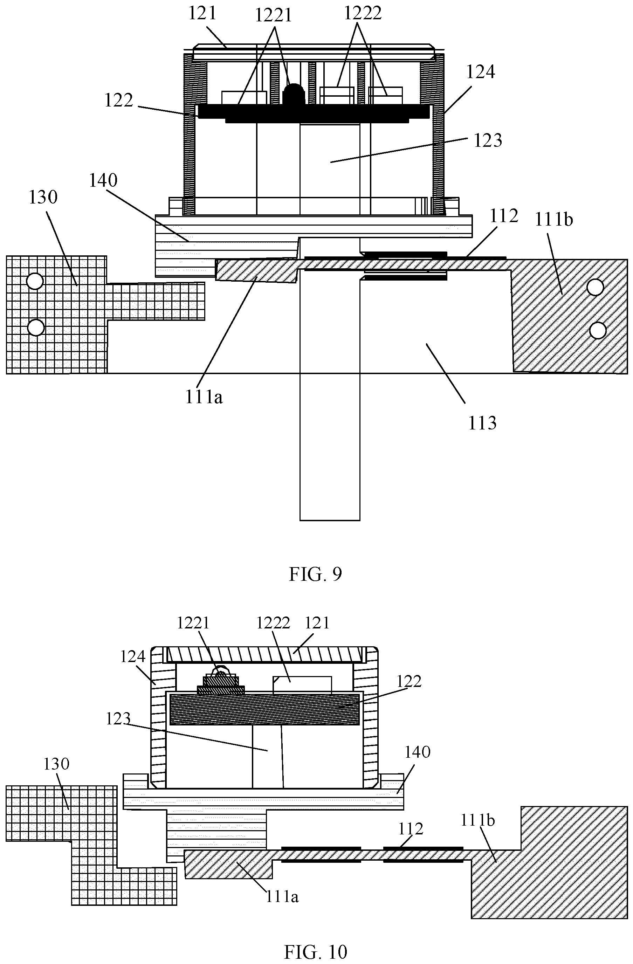

Specifically, as shown in FIG. 9 and FIG. 10, the PPG detection module 120 may include: a light-transmitting cover 121, a circuit board 122 and a second connecting member 123.

The light-transmitting cover 121 is configured to receive the pressing of the user and may be a cover prepared by a transparent material. For example, the transparent material may be a material with high light transmittance such as glass or resin so as to reduce the attenuation of the optical signal when passing through the light-transmitting cover 121.

A light source 1221 and a light detector 1222 are welded on the circuit board 122. When the finger of the user presses the light-transmitting cover 121, the light source 1221 is used to emit an optical signal to the finger of the user, and the light detector 1222 is configured to receive a first optical signal that is emitted by the light source and returned via reflection or transmission of the finger and is configured to convert the optical signal into the PPG signal.

The light source 1221 may include, but is not limited to a point light source, for example, a light-emitting diode (LED), a laser diode (LD) or an infrared emitting diode; and the light source 1221 may also be a linear light source or a planar light source, which is not specifically limited by the embodiment of the present application.

Optionally, the light source 1221 may include one light source (as shown in FIG. 10) or a plurality of light sources (as shown in FIG. 9), which may emit optical signals of one or more wavebands. For example, the light source 1221 may emit the optical signal of a red light waveband.

In a case where the light source 1221 includes a plurality of light sources, the plurality of light sources may emit the optical signals to the finger of the user in a time-shared manner or may emit the optical signals to the finger of the user at the same time, and the plurality of light sources may emit the optical signal of the same waveband or may emit the optical signals of different wavebands. For example, the light source 1221 includes a light source 1221 and a light source 1221b, where the light source 1221a may emit the optical signal of the red light waveband at a first time, and the light source 1221b may emit the optical signal of a green light waveband at a second time.

A plurality of light sources are provided to solve the problem that the PPG signal for detecting the biological characteristic information cannot be acquired due to that the optical signal emitted by single light source cannot arrive at the finger of the user or the quantity of the signal arriving at the finger of the user is small. Further, the plurality of light sources are set to emit the optical signals of different wavebands, so that the technical solution of the embodiment of the present application can detect the biological characteristic information according to the PPG signals of different wavebands, and the accuracy of detecting the biological characteristic information can be improved.

The light detector 1222 may include, but is not limited to a photodiode (PD), a phototriode and the like. Further, the light detector 1222 may further include an optical component. As an example, the optical component may be arranged above the PD and is configured to guide more effective optical signals to enter the PD so as to improve the optical detection efficiency of the PD.

Optionally, the light detector 1222 may further include one (shown in FIG. 10) or a plurality of light detectors (shown in FIG. 9). For example, the light detector 1222 may include a plurality of PDs. At this time, the light detector 1222 may be a PD array.

According to the above technical solution, a plurality of light detectors are provided, so that the plurality of light detectors may receive a first optical signal, the first optical signal received by the plurality of light detectors is used to form a plurality of PPG signals, and the plurality of PPG signals are used to comprehensively detect the biological characteristic information of the user, so that the accuracy of detecting the biological characteristic information can be improved.

Optically, the circuit board 122 may be a printed circuit board (PCB) or FPC and is configured to being electrically connected to the light source 1221 and the light detector 1222.

The circuit board is set as the FPC. The occupied space of the FPC is smaller relative to the PCB, so the detection device can be better applied to the small electronic device (such as a smartphone).

When the circuit board 122 is the FPC, the detection device may further include a conductive reinforcement, where the conductive reinforcement is fixed at the bottom of the FPC and configured to support the FPC. Optionally, the conductive reinforcement may be a reinforced steel sheet. At present, the reinforcement used by most circuit board manufacturers is the reinforced steel sheet. According to the above technical solution, the conductive reinforcement is set as the reinforced steel sheet, and the technical process of the circuit board manufacturers is changed minimally.

Optionally, a shape of the light-transmitting cover 121 may be a waist circle. Due the large area of the waist circle, the light-transmitting cover is set as the waist circle, so that more light sources and light detectors may be welded on the circuit board, for example, double lamps and double PDs shown in FIG. 9, so that as mentioned above, the accuracy of detecting the biological characteristic information can be improved.

Or a shape of the light-transmitting cover 121 may be a circle. At this time, exemplarily, as shown in FIG. 10, there may be one light source 1221 and one light detector 1222. Since the area of the circle is small relative to the waist circle, the shape of the light-transmitting cover is set as a circle, and the size of the detection device can be further reduced.

Of course, the shape of the light-transmitting cover 121 may be other regular or irregular shapes, which is not specifically limited by the embodiment of the present application.

Optionally, one end of the second connecting member 123 may be connected to the circuit board 122, and the other end of the second connecting member 123 may be connected to the main board of the electronic device, for transmitting the PPG signal to the main board of the electronic device. Similar to the first connecting member 114, the second connecting member 123 may also be an FPC or cable.

Further, continuously referring to FIG. 9 and FIG. 10, the PPG detection module 120 may further include: a first holder 124, where an upper surface of the first holder 124 is fixedly connected to the light-transmitting cover 121.

Optionally, the light-transmitting cover 121 may be fixedly connected to the first holder 124 through a second glue layer. The second glue layer, for example, may be organic solid glue with high light transmittance. The light-transmitting cover is connected to the first holder through the second glue layer, which is beneficial to realize the miniaturization of the detection device. Further, the second glue layer has light-transmitting property, so that the optical signal penetrating the light-transmitting cover can arrive at the light detector.

Of course, the light-transmitting cover 121 may also be fixedly connected to the first holder 124 through other modes. For example, the light-transmitting cover may be fixedly connected to the first holder through screws.

In one implementation manner, the first holder 124 is internally provided with a first cavity, and the circuit board 122 and at least part of the second connecting member 123 are arranged in the first cavity. The first cavity may be filled with air, thereby having little influence on the transmission of the optical signal; or the first cavity may also be filled with a transparent medium layer with high light transmittance, thereby improving the overall stability of the detection device.

In this implementation manner, the first holder 124 may be of a sleeve structure.

Optionally, the circuit board 122 and at least part of the second connecting member 123 may be fixedly connected to the first holder 124 through a third glue layer. Similar to the second glue layer, the circuit board and at least part of the second connecting member are connected to the first holder through the third glue layer, thereby realizing the miniaturization of the detection device.

According to the above technical solution, by the first holder, the circuit board and the light-transmitting cover can be fixed, and an up-down position relation between the PPG detection module and the electronic device may be flexibly adjusted by adjusting a height of the first holder.

It should be noted that the PPG detection module described above is fixed on the upper surface of the supporting member 140, specifically for the lower surface of the first holder 124 may be fixed on the upper surface of the supporting member 140.

In another implementation manner, the first holder 124 may be fixed on the circuit board 122. In this implementation manner, the first holder 124 may be of a ring structure, that is, the first holder 124 may be of a metal structure.

According to the above technical solution, the first holder is arranged on the circuit board, which is beneficial to protect the light-transmitting cover of the PPG detection module, the light source and the light detector. Furthermore, the first holder can play a decorative role.

Optionally, the shape of the first holder 124 may be the same as or similar to the shape of the light-transmitting cover 121. For example, when the shape of the light-transmitting cover 121 is a waist circle, the shape of the first holder 124 may also be a waist circle; and when the shape of the light-transmitting cover 121 is a circle, the shape of the first holder 124 may also be a circle.

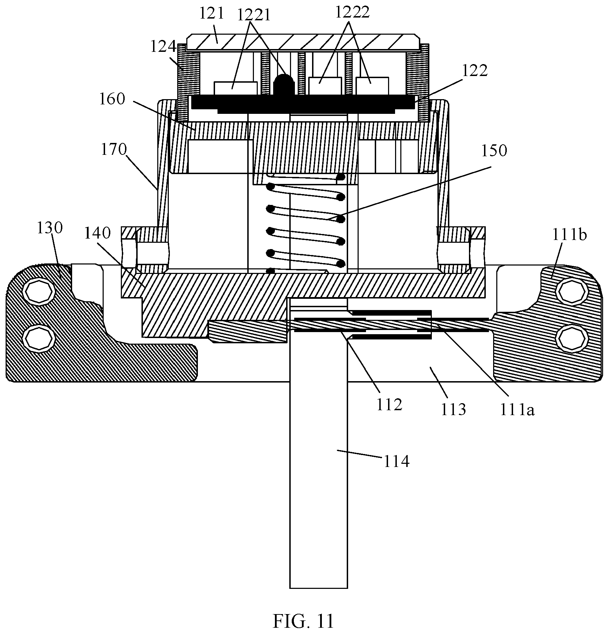

In general, the pressing pressure required for the biological characteristic information detection needs to be within a certain range, so the pressing pressure is not as large as possible or as small as possible. For this problem, as shown in FIG. 11, the detection device 100 may further include an elastic member 150, where the elastic member 150 is arranged below the PPG detection module 120 and configured to limit a movable distance of the PPG detection module 120 within a preset range so as to limit a force applied by the user within a pressure range required by the biological characteristic information detection.

Optionally, the elastic member 150 may be a spring or any other type of elastic component, such as a part made of an elastic rubber material, which is not specifically limited by the embodiment of the present application.

cation.

According to the embodiment of the present application, an elastic force of the elastic member may be controlled within the pressure range required for the biological characteristic information detection by controlling an elastic parameter of the elastic member 150, that is, the pressing pressure of pressing the electronic device by the user may be prevented from being too large or too small, so that the biological characteristic information can be detected accurately. Further, damage to the detection device caused by excessive pressing pressure of the user may be avoided.

Specifically, when the user presses the electronic device, that is, the user presses the light-transmitting cover 121, the PPG detection module 120 deforms downwards and compresses the elastic member 150, the elastic member 150 generates a corresponding elastic force and acts on the pressure detection module 110, and the pressure detection module 110 may detect the pressure signal applied to the electronic device by the user. With the increase of the pressing pressure of the user, the elastic member 150 is gradually compressed until a maximum compression quantity is reached, the elastic member 150 is no longer compressed, and the user will stop increasing the pressing force when feeling that the user cannot continue to press downwards.

Or if the pressing pressure of pressing the electronic device by the user is too small and is not in the pressure range required for the biological characteristic detection, the elastic member 150 will not compress, and the user will increase the pressing pressure based on the previous pressing when feeling that the detection device 100 does not move downwards.

However, when the user presses the electronic device, there may be a problem that the elastic member 150 is skewed. Therefore, as shown in FIG. 11, the elastic member 150 may be fixedly connected to the PPG detection module 120 through the sliding holder 160. That is, the PPG detection module 120 is fixed on the sliding holder 160, and the elastic member 150 is arranged below the sliding holder 160.

Optionally, a material for manufacturing the sliding holder 160 may be a self-lubricating material, for example, brass, polyamide (PA) and the like.

Since the sliding holder has a good guide function, the sliding holder is arranged between the elastic member and the PPG detection module to avoid the problem that the elastic member is skewed when the user presses the electronic device, so that the elastic member may slide better in the second direction, where the second direction is vertical to the elastic arm 111a.

Further, continuously referring to FIG. 11, the detection device further includes a second holder 170, where the second holder 170 is fixedly connected to the sliding holder 160, the second holder 170 is internally provided with a second cavity, and the sliding holder 160 and the elastic member 150 can slide in the second cavity in the second direction.

Optionally, similar to the first holder 124, the second holder 170 may also be of a sleeve structure.

Optionally, a lower surface of the second holder 170 may be fixed on the supporting member 140. For example, the second holder 170 may be fixed on the supporting member 140 through a dispensing mode, or the second holder may be fixed on the supporting member 140 through a mechanical mode (such as screw).

It may be seen from FIG. 11 that a gap is formed between the second holder 170 and the sliding holder 160. Preferably, the gap between the second holder 170 and the sliding holder 160 may be 30μm-50μm. In this way, the sliding holder has a better guide function.

Optionally, a size of the second holder 170 in the first direction may be equal to a size of the PPG detection module 120 in the first direction.

Or as shown in FIG. 11, the size of the second holder 170 in the first direction may be greater than the size of the PPG detection module 120 in the first direction.

The size of the second holder in the first direction is set to be greater than the size of the PPG detection module in the first direction, that is, the PPG detection module is wrapped in the second holder, so that the PPG detection module may be prevented from outwards slipping off in the sliding process.

Further, a stop block may be arranged at the top of the second holder 170 so as to prevent the sliding holder 160 from upwards slipping off when the elastic member 150 springs back to the original position.

Exemplarily, the stop block may be stepped, or may be T-shaped, or may also be convex.

According to the above technical solution, a second holder is provided to further prevent the elastic member from wobbling in a first direction in the sliding process, thereby ensuring the accuracy of the pressure signal detected by the pressure detection module and ensure the accuracy of detecting the biological characteristic information.

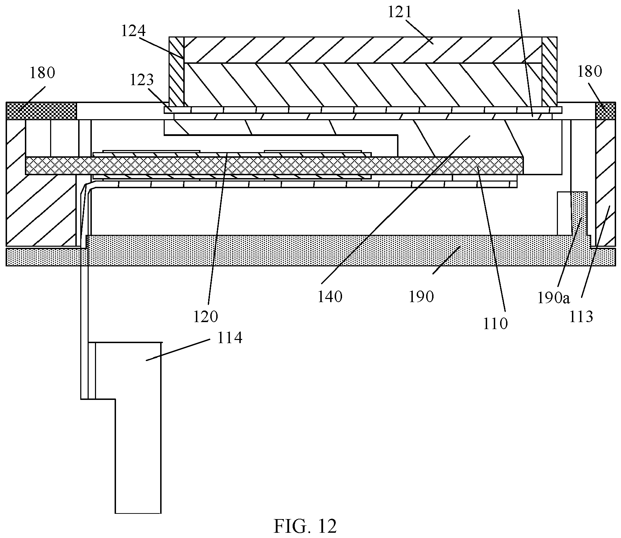

In daily life, the electronic device may be in various humid environments. Based on this problem, as shown in FIG. 12, the detection device 100 may further include a first sealing member 180, where the first sealing member 180 may adhere to a circle around an upper surface of the module holder 113. The first sealing member 180, for example, may be a soft rubber sealing member, such as a silica gel ring and a rubber ring.

By the first sealing member, the waterproof function of the detection device may be realized.

When the electronic device is a smartphone, the first sealing member 180 may further cooperate with a middle frame of the smartphone so as to effectively realize the waterproof function of the detection device 100. For example, if the first sealing member 180 is the silica gel ring, the silica gel ring may be pressed on the middle frame.

Optionally, the first sealing members 180 have different sizes at both ends of the first direction. Referring to FIG. 12 again, it may be seen from FIG. 12 that the size of the first sealing member 180 on the left side is greater than the size of the sealing member 180 on the right side.

Moreover, the detection device 100 may further include a second sealing member 190, where the second sealing member 190 may be arranged at the periphery of a lower surface of the module holder 113. Exemplarily, the second sealing member 190 may be a plastic sealing member or a metal sealing member.

According to the above technical solution, the second sealing member is provided while the first sealing member is provided, and the second sealing member is arranged in a direction opposite to the first sealing member, so that the first sealing member and the second sealing member may realize the waterproof function in different directions, that is, the second sealing member may assist the first sealing member in preventing water, so that the waterproof function of the detection device can be further improved.

In some designs, the second sealing member 190 not only can realize the waterproof function, but also can realize the function of the stop block 130 described above. Specifically, as shown in FIG. 12, the second sealing member 190 is provided with an upward protrusion 119a on one side away from the fixed base 111b in the first direction, and the protrusion 119a may at least partially overlap the elastic arm 111a in the first direction so as to avoid serious deformation of the cantilever beam 111.

In this design, the detection device does not need to be additionally provided with the protective stop block, which is beneficial to further reduce the size and cost of the detection device.

Optionally, in the embodiment of the present application, the detection device 100 may be arranged on any surface of the electronic device.

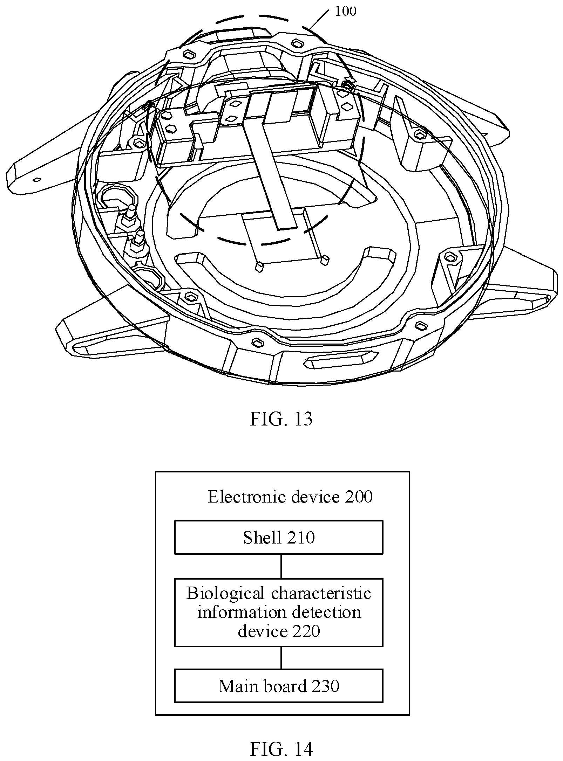

In one implementation manner, the detection device 100 may be arranged on a side of the electronic device. As shown in FIG. 13, the electronic device is a watch and the detection device 100 is arranged on a side of the watch.

In this implementation manner, the electronic device may be specially provided with a button for detecting the biological characteristic information, and the button may be a light-transmitting cover 121.

Or the detection device 100 may multiplex other function buttons on the side of the electronic device. For example, if the electronic device is a mobile phone, the detection device 100 may multiplex a power button, a fingerprint button or a volume button on the side of the mobile phone; and if the electronic device is a watch, the detection device may multiplex a button for adjusting time of the watch or a power button.

A plurality of functions of the electronic device are integrated into the same button, which facilitates the miniaturization development and design of the electronic device, is favorable for the production and manufacturing of the electronic device, reduces the manufacturing process and the manufacturing cost, and is also favorable for improving the appearance of the electronic device.

According to the above technical solution, the detection device is arranged on the side of the electronic device, so that additional space on the front side or back side of the electronic device can be prevented from being occupied, and the aesthetic degree of the electronic device can be increased. Furthermore, if the electronic device is a wearable device, for example, a smartwatch and the like, the detection device is arranged on the side of the smartwatch, which does not affect the wearing experience on the back side of the watch and affect the screen display on the front side of the watch and makes the appearance of the watch more fashionable and beautiful, so that the user experience can be improved.

In another implementation manner, the detection device 100 may be arranged on a back side or front side of the electronic device. If the detection device 100 is arranged on the back side of the electronic device, the "upper" of the detection device 100 is the "lower" of the electronic device and the "lower" of the detection device 100 is the "upper" of the electronic device; and if the detection device 100 is arranged on the front side of the electronic device, the detection device 100 and the electronic device have the same direction.

Optionally, the light-transmitting cover 121 of the detection device 100 may be arranged in a fingerprint identification region of the electronic device, so that the biological characteristic information may be detected while the user unlocks the electronic device. For example, if the detection device 100 is arranged on the front side of the electronic device, the light-transmitting cover 121 may multiplex a display screen of the electronic device so as to reduce the production cost of the detection device 100.

Optionally, in the embodiment of the present application, the detection device 100 may further include a processing unit for calculating the biological characteristic information of the user according to the PPG signal and the pressure signal. Or the electronic device may include a processing unit for calculating the biological characteristic information of the user according to the received PPG signal and pressure signal.

The processing unit may be a central processing unit (CPU). The processor may also be other general processors, digital signal processor (DSP), application specific integrated circuit (ASIC), field programmable gate array (FPGA) or other programmable logic devices, discrete gates or transistor logic devices, discrete hardware components, and the like. The general processor may be a microprocessor, or the processor may also be any conventional processor.

After the processing unit acquires the biological characteristic information of the user, the processing unit may prompt the user that the detection is completed, so that the user does not press the electronic device any more. Furthermore, the electronic device may output the biological characteristic information of the user. For example, the electronic device may display blood pressure values of users on the display screen. Further, considering that some users are unclear about the normal value range of the biological characteristic information, the electronic device may also prompt the user whether the biological characteristic information at the current time is normal. For example, if the blood pressure of the user is 110 millimeters of mercury (mmHg), the display screen may display the word "normal" or the expression of smiling face or the animation of thumbs up. Or the electronic device may broadcast the blood pressure of the user by voice and broadcast whether the blood pressure of the user is normal by voice, for example, "your blood pressure is normal". Or the electronic device may broadcast the blood pressure of the user while displaying the blood pressure of the user.

The biological characteristic information detection device provided by the embodiment of the present application adopts the pressure detection module and the PPG detection module, acquires the pressure signal applied to the electronic device by the user when the user actively presses the electronic device and the PPG signal at the pressing part of the user, and then detects the biological characteristic information of the user in combination with the pressure signal and the PPG signal. Relative to detecting the biological characteristic information of the user by single parameter, the detection device provided by the embodiment of the present application can effectively improve the detection precision of the biological characteristic information. In addition, the pressure detection module and the PPG detection module are stacked. When the user presses the electronic device, the pressure signal detected by the pressure detection module and the PPG signal detected by the PPG detection module are a pressure signal and a PPG signal at the same part of the finger of the user, that is, the pressure signal and the PPG signal have higher correspondence. The biological characteristic information of the user detected by the pressure signal and the PPG signal has higher accuracy.

Further, the simple cantilever structure cooperates with the pressure sensor to form the pressure detection module. The cantilever beam structure is small, so the detection device provided by the embodiment of the present application has the advantage of miniaturization, can be integrated in the electronic device, and is convenient for users to carry.

The embodiment of the present application further provides an electronic device. As shown in FIG. 14, the electronic device 200 may include a shell 210, a biological characteristic information detection device 220 and a main board 230.

The biological characteristic information detection device 220 may the biological characteristic information detection device 100 in the above embodiments, and at least part of the biological characteristic information detection device 220 may be arranged in the shell 210. For example, the pressure detection module (such as the pressure detection module 110 in the above embodiments) in the biological characteristic information detection device 220 may be arranged in the shell 210.

The main board 230 is arranged in the shell 210 and is electrically connected to the biological characteristic information detection device 220. For example, the first connecting member (such as the first connecting member 114 in the PPG detection module in the above embodiments) in the biological characteristic information detection device 220 is electrically connected to the main board 230, and the second connecting member (such as the second connecting member 123 in the PPG detection module in the above embodiments) in the biological characteristic information detection device 220 is electrically connected to the main board 230.

Optionally, the electronic device 200 may further include a button; the button may include a cavity; and the PPG detection module (such as the PPG detection module 120 in the above embodiments) in the biological characteristic information detection device 220 may be arranged in the cavity, or the biological characteristic information detection device 220 may be entirely arranged in the cavity.

As an example rather than a limitation, the electronic device in the embodiment of the present application may be smartwatch or a smartphone. The detection device provided by the present application is convenient to carry, has high detection accuracy and is arranged in the watch and the mobile phone, so that the user may realize biological characteristic information detection anytime anywhere conveniently through the mobile phone or watch carried by the user, the biological characteristic information detection is no longer limited to medical devices, and the biological characteristic information detection can be better popularized and serve people's daily life.

## Claims

1. A biological characteristic information detection device (100) for being arranged on an electronic device, wherein the detection device comprises:

   a pressure detection module (110) comprising a cantilever beam (111) and a pressure sensor (112), wherein the pressure sensor (112) is fixed on a surface of the cantilever beam (111), the cantilever beam (111) comprises an elastic arm (111a) and a fixed base (111b), the fixed base (111b) is fixed in the electronic device, and the elastic arm (111a) is suspended in the air; and a photoplethysmography (PPG) detection module (120) arranged on the elastic arm (111a) and configured to detect a PPG signal of a user when the user presses the electronic device, wherein when the user presses the electronic device, a pressure signal applied to the electronic device by the user is transmitted to the cantilever beam (111) through the PPG detection module (120), so that the pressure sensor (112) detects the pressure signal, and the pressure signal and the PPG signal are used to detect biological characteristic information of the user; the biological characteristic information detection device (100) further comprising:

   an elastic member (150) arranged on one opposite side of the PPG detection module (120) facing towards the electronic device for sensing a pressing direction of the user and configured to limit a movable distance of the PPG detection module (120) within a preset range so as to limit a force applied by the user within a pressure range required by the biological characteristic information detection, and an elastic force of the elastic member (150) is controlled within the pressure range required for the biological characteristic information detection by controlling an elastic parameter of the elastic member (150); wherein the elastic member (150) is fixedly connected to the PPG detection module (120) through a sliding holder (160); the detection device further comprising: a second holder (170) fixedly connected to the sliding holder (160), the second holder (170) being internally provided with a second cavity, the sliding holder (160) and the elastic member (150) sliding in the second cavity along a second direction, and the second direction being perpendicular to the elastic arm (111a).

2. The detection device according to claim 1, wherein a thickness of the cantilever beam (111) is 0.1mm-0.6mm; and the cantilever beam (111) is an integrated structure and the elastic arm (111a) and the fixed base (111b) are integrally connected or the cantilever beam (111) is a split structure, and the elastic arm (111a) is fixedly connected to the fixed base (111b).

3. The detection device according to claim 1 or 2, wherein the pressure sensor (112) comprises four strain gauges, the four strain gauges forming a full-bridge circuit; and two strain gauges of the four strain gauges are fixed on one side of the elastic arm (111a) facing towards the electronic device for sensing pressing of the user, and the other two strain gauges are fixed on the other side of the elastic arm (111a).

4. The detection device according to any one of claims 1 to 3, wherein the pressure detection module (110) further comprises:
a protective stop block (130) arranged on one side of the elastic arm (111a) away from the fixed base (111b) in a first direction and at least partially overlapping with the elastic arm (111a) in the first direction, the first direction being a direction parallel with the elastic arm (111a).

5. The detection device according to any one of claims 1 to 4, wherein the pressure detection module (110) further comprises:
a first connecting member (114), one end of the first connecting member (114) being connected to the cantilever beam (111) and the other end of the first connecting member (114) being connected to a main board of the electronic device, and the pressure detection module (110) transmitting an electrical signal corresponding to the pressure signal to the main board of the electronic device through the first connecting member (114).

6. The detection device according to any one of claims 1 to 5, wherein the PPG detection module (120) sequentially comprises a light-transmitting cover (121), a circuit board (122) and a second connecting member (123) from one side facing towards the electronic device for sensing the pressing of the user to the opposite side;

the light-transmitting cover (121) is configured to receive the pressing of the user;
a light source (1221) and a light detector (1222) are welded on the circuit board (122), the light source (1221) is used to emit an optical signal to a finger of the user, and the light detector (1222) is configured to receive a first optical signal that is emitted by the light source (1221) and returned via reflection or transmission of the finger and convert the first optical signal into the PPG signal;
the second connecting member (123) is configured to transmit the PPG signal to the main board of the electronic device.

7. The detection device according to claim 6, wherein the PPG detection module (120) further comprises:

a first holder (124), one side of the first holder (124) facing towards the electronic device for sensing the pressing of the user being fixedly connected to the light-transmitting cover (121), the first holder (124) being internally provided with a first cavity, and at least part of the second connecting member (123) being arranged in the first cavity; and
the circuit board (122) is arranged in the first cavity, and the circuit board (122) and at least part of the second connecting member (123) are fixedly connected to the first holder (124) through a glue layer.

8. The detection device according to any one of claims 1 to 7, wherein a gap between the sliding holder (160) and the second holder (170) is 30μm-50μm.

9. The detection device according to any one of claims 1 to 7, wherein a stop block is arranged at a top of one side of the second holder (170) facing towards the electronic device for sensing the pressing of the user.

10. The detection device according to any one of claims 1 to 9, wherein a size of the second holder (170) in a first direction is greater than a size of the PPG detection module (120) in the first direction, the first direction being perpendicular to the second direction.

11. The detection device according to any one of claims 1 to 10, wherein the pressure detection module (110) further comprises:

a module holder (113), the fixed base (111b) being fixed in the electronic device through the module holder (113);
a first sealing member (180) adhering to a circle around one side of the module holder (113) facing towards the electronic device for sensing the pressing of the user; and
a second sealing member (190) arranged on one side of the module holder (113) opposite to the first sealing member (180).

12. An electronic device, comprising:

a shell;
the biological characteristic information detection device (100) according to any one of claims 1 to 11, the biological characteristic information detection device being at least partially arranged in the shell; and
a main board arranged in the shell and electrically connected to the biological characteristic information detection device (100).

**Patentansprüche**

1. Vorrichtung (100) zur Detektion von Informationen über biologische Merkmale zur Anordnung auf einer elektronischen Vorrichtung, wobei die Detektionsvorrichtung umfasst:

ein Druckerfassungsmodul (110), das einen freitragenden Träger (111) und einen Drucksensor (112) umfasst, wobei der Drucksensor (112) auf einer Oberfläche des freitragenden Trägers (111) befestigt ist, der freitragende Träger (111) einen elastischen Arm (111a) und eine feste Basis (111b) umfasst, die feste Basis (111b) in der elektronischen Vorrichtung befestigt ist und der elastische Arm (111a) in der Luft schwebt; und ein Photoplethysmographie (PPG)-Erfassungsmodul (120), das auf dem elastischen Arm (111a) angeordnet und so konfiguriert ist, dass es ein PPG-Signal eines Benutzers erfasst, wenn der Benutzer die elektronische Vorrichtung drückt,

wobei, wenn der Benutzer die elektronische Vorrichtung drückt, ein Drucksignal, das von dem Benutzer auf die elektronische Vorrichtung ausgeübt wird, durch das PPG-Erfassungsmodul (120) an den freitragenden Träger (111) übertragen wird, so dass der Drucksensor (112) das Drucksignal erfasst, und das Drucksignal und das PPG-Signal verwendet werden, um biologische charakteristische Informationen des Benutzers zu erfassen,

wobei die Vorrichtung (100) zur Detektion von Informationen über biologische Merkmale weiterhin umfasst:

ein elastisches Element (150), das angeordnet auf einer gegenüberliegenden Seite des PPG-Detektionsmoduls (120) der elektronischen Vorrichtung zugewandt ist, um eine Druckrichtung des Benutzers zu erfassen, und das so konfiguriert ist, dass es einen bewegbaren Abstand des PPG-Detektionsmoduls (120) innerhalb eines voreingestellten Bereichs begrenzt, um eine vom Benutzer ausgeübte Kraft innerhalb eines Druckbereichs zu begrenzen, der für die Detektion von Informationen über biologische Merkmale erforderlich ist, und wobei eine elastische Kraft des elastischen Elements (150) innerhalb des für die Detektion von Informationen über biologische Merkmale erforderlichen Druckbereichs durch Steuern eines elastischen Parameters des elastischen Elements (150) gesteuert wird; wobei das elastische Element (150) über eine Schiebehalterung (160) fest mit dem PPG-Erfassungsmodul (120) verbunden ist;

wobei die Detektionsvorrichtung ferner umfasst: eine zweite Halterung (170), die fest mit der Schiebehalterung (160) verbunden ist, wobei die zweite Halterung (170) im Inneren mit einem zweiten Hohlraum versehen ist, wobei die Schiebehalterung (160) und das elastische Element (150) in dem zweiten Hohlraum entlang einer zweiten Richtung gleiten, wobei die zweite Richtung senkrecht zu dem elastischen Arm (111a) ist.

2. Detektionsvorrichtung nach Anspruch 1, wobei die Dicke des freitragenden Trägers (111) 0,1 mm bis 0,6 mm beträgt; und
der freitragende Träger (111) eine integrierte Struktur ist und der elastische Arm (111a) und die feste Basis (111b) fest verbunden sind oder der freitragende Träger (111) eine geteilte Struktur ist und der elastische Arm (111a) fest mit der festen Basis (111b) verbunden ist.

3. Detektionsvorrichtung nach Anspruch 1 oder 2, wobei der Drucksensor (112) vier Dehnungsmessstreifen umfasst, wobei die vier Dehnungsmessstreifen eine Vollbrückenschaltung bilden; und zwei Dehnungsmessstreifen der vier Dehnungsmessstreifen auf einer Seite des elastischen Arms (111a) befestigt sind, die der elektronischen Vorrichtung zugewandt ist, um das Drücken des Benutzers zu erfassen, und die anderen zwei Dehnungsmessstreifen auf der anderen Seite des elastischen Arms (111a) befestigt sind.

4. Detektionsvorrichtung nach einem der Ansprüche 1 bis 3, wobei das Druckerfassungsmodul (110) ferner umfasst:
einen schützenden Sperrblock (130), der auf einer Seite des elastischen Arms (111a) entfernt von der festen Basis (111b) in einer ersten Richtung angeordnet ist und den elastischen Arm (111a) in der ersten Richtung zumindest teilweise überlappt, wobei die erste Richtung eine Richtung parallel zu dem elastischen Arm (111a) ist.

5. Detektionsvorrichtung nach einem der Ansprüche 1 bis 4, wobei das Druckerfassungsmodul (110) ferner umfasst:
ein erstes Verbindungselement (114), wobei ein Ende des ersten Verbindungselements (114) mit dem freitragenden Träger (111) verbunden ist und das andere Ende des ersten Verbindungselements (114) mit einer Hauptplatine der elektronischen Vorrichtung verbunden ist, und das Druckerfassungsmodul (110) ein elektrisches Signal, das dem Drucksignal entspricht, über das erste Verbindungselement (114) an die Hauptplatine der elektronischen Vorrichtung überträgt.

6. Detektionsvorrichtung nach einem der Ansprüche 1 bis 5, wobei das PPG-Detektionsmodul (120) aufeinander folgend eine lichtdurchlässige Abdeckung (121), eine Leiterplatte (122) und ein zweites Verbindungselement (123) umfasst, das von einer Seite aus der elektronischen Vorrichtung zugewandt ist, um den Druck des Benutzers auf die gegenüberliegende Seite zu erfassen;

die lichtdurchlässige Abdeckung (121) so konfiguriert ist, dass sie das Drücken des Benutzers aufnimmt;

eine Lichtquelle (1221) und ein Lichtdetektor (1222) auf die Leiterplatte (122) geschweißt sind, wobei die Lichtquelle (1221) verwendet wird, um ein optisches Signal an einen Finger des Benutzers zu emittieren, und der Lichtdetektor (1222) so konfiguriert ist, dass er ein erstes optisches Signal empfängt, das von der Lichtquelle (1221) emittiert wird und durch Reflexion oder Transmission des Fingers zurückkommt, und das erste optische Signal in das PPG-Signal umwandelt;

das zweite Verbindungselement (123) so konfiguriert ist, dass es das PPG-Signal an die Hauptplatine der elektronischen Vorrichtung überträgt.

7. Detektionsvorrichtung nach Anspruch 6, wobei das PPG-Detektionsmodul (120) weiterhin umfasst:

eine erste Halterung (124), wobei eine Seite der ersten Halterung (124), die der elektronischen Vorrichtung zum Erfassen des Drückens des Benutzers zugewandt ist, fest mit der lichtdurchlässigen Abdeckung (121) verbunden ist, wobei die erste Halterung (124) integriert mit einem ersten Hohlraum versehen ist und zumindest ein Teil des zweiten Verbindungselements (123) in dem ersten Hohlraum angeordnet ist; und die Leiterplatte (122) ist in dem ersten Hohlraum angeordnet, und die Leiterplatte (122) und mindestens ein Teil des zweiten Verbindungselements (123) mit der ersten Halterung (124) durch eine Klebeschicht fest verbunden sind.

8. Detektionsvorrichtung nach einem der Ansprüche 1 bis 7, wobei ein Spalt zwischen der Schiebehalterung (160) und der zweiten Halterung (170) 30$\mu$m-50$\mu$m beträgt.

9. Detektionsvorrichtung nach einem der Ansprüche 1 bis 7, wobei an der Oberseite einer der elektronischen Vorrichtung zugewandten Seite der zweiten Halterung (170) ein Sperrblock angeordnet ist, um das Drücken des Benutzers zu erfassen.

10. Detektionsvorrichtung nach einem der Ansprüche 1 bis 9, wobei eine Größe der zweiten Halterung (170) in einer ersten Richtung größer ist als eine Größe des PPG-Detektionsmoduls (120) in der ersten Richtung, wobei die erste Richtung senkrecht zu der zweiten Richtung verläuft.

11. Detektionsvorrichtung nach einem der Ansprüche 1 bis 10, wobei das Druckerfassungsmodul (110) ferner umfasst:

einen Modulhalter (113), wobei die feste Basis (111b) durch den Modulhalter (113) in der elektronischen Vorrichtung befestigt ist;

ein erstes Dichtungselement (180), das an einem Kreis um eine Seite des Modulhalters (113) haftet, die der elektronischen Vorrichtung zugewandt ist, um den Druck des Benutzers zu erfassen; und

ein zweites Dichtungselement (190), das auf einer Seite des Modulhalters (113) gegenüber dem ersten Dichtungselement (180) angeordnet ist.

12. Elektronische Vorrichtung umfassend:

ein Gehäuse;

die Vorrichtung (100) zur Detektion von Informationen über biologische Merkmale nach einem der Ansprüche 1 bis 11, wobei die Vorrichtung zur Detektion von Informationen über biologische Merkmale zumindest teilweise in dem Gehäuse angeordnet ist; und

eine Hauptplatine, die in dem Gehäuse angeordnet und elektrisch mit der Vorrichtung (100) zur Detektion von Informationen über biologische Merkmale verbunden ist.

**Revendications**

1. Dispositif de détection d'informations sur des composantes biologiques (100) destiné à être agencé sur un dispositif électronique, dans lequel le dispositif de détection comprend :

un module de détection de pression (110) comprenant un profilé en porte-à-faux (111) et un capteur de pression (112), dans lequel le capteur de pression (112) est fixé sur une surface du profilé en porte-à-faux (111), le profilé en porte-à-faux (111) comprenant un bras élastique (111a) et une base fixe (111b), la base fixe (111b) étant fixée dans le dispositif électronique, et le bras élastique (111a) étant suspendu dans l'air ; et

un module de détection de photopléthysmographie (PPG) (120) agencé sur le bras élastique (111a) et configuré pour détecter un signal de PPG d'un utilisateur lorsque l'utilisateur appuie sur le dispositif électronique,

dans lequel, lorsque l'utilisateur appuie sur le dispositif électronique, un signal de pression appliqué au dispositif électronique par l'utilisateur est transmis au profilé en porte-à-faux (111) à travers le module de détection de PPG (120), de sorte que le capteur de pression (112) détecte le signal de pression, et le signal de pression et le signal de PPG sont utilisés pour détecter des informations sur des composantes biologiques de l'utilisateur ;

le dispositif de détection d'informations sur des composantes biologiques (100) comprenant en outre :

un élément élastique (150) agencé sur un côté opposé du module de détection de PPG (120) faisant face au dispositif électronique pour détecter une direction de pression de l'utilisateur et configuré pour limiter une distance mobile du module de détection de PPG (120) au sein d'une plage prédéfinie de façon à limiter une force appliquée par l'utilisateur dans une plage de pression requise par la détection d'informations sur des composantes biologiques, et une force élastique de l'élément élastique (150) est commandée au sein de la plage de pression requise pour la détection d'informations sur des composantes biologiques en commandant un paramètre élastique de l'élément élastique (150) ;

dans lequel l'élément élastique (150) est connecté de manière fixe au module de détection de PPG (120) par l'intermédiaire d'un support coulissant (160) ;

le dispositif de détection comprenant en outre : un second support (170) connecté de manière fixe au support coulissant (160), le second support (170) étant pourvu à l'intérieur d'une seconde cavité, le support coulissant (160) et l'élément élastique (150) coulissant dans la seconde cavité le long d'une seconde direction, et la seconde direction étant perpendiculaire au bras élastique (111a).

2. Dispositif de détection selon la revendication 1, dans lequel une épaisseur du profilé en porte-à-faux (111) est comprise entre 0,1 mm et 0,6 mm ; et

le profilé en porte-à-faux (111) est une structure intégrée et le bras élastique (111a) et la base fixe (111b) sont connectés d'un seul tenant, ou le profilé en porte-à-faux (111) est une structure divisée et le bras élastique (111a) est connecté de manière fixe à la base fixe (111b).

3. Dispositif de détection selon la revendication 1 ou 2, dans lequel le capteur de pression (112) comprend quatre jauges de contrainte, les quatre jauges de contrainte formant un circuit en pont complet ; et

deux jauges de contrainte sur les quatre jauges de contrainte sont fixées sur un côté du bras élastique (111a) faisant face au dispositif électronique de détection d'une pression de l'utilisateur, et les deux autres jauges de contrainte sont fixées sur l'autre côté du bras élastique (111a).

4. Dispositif de détection selon l'une quelconque des revendications 1 à 3, dans lequel le module de détection de pression (110) comprend en outre :

un bloc d'arrêt protecteur (130) agencé sur un côté du bras élastique (111a) à l'opposé de la base fixe (111b), dans une première direction et chevauchant au moins partiellement le bras élastique (111a) dans la première direction, la première direction étant une direction parallèle au bras élastique (111a).

5. Dispositif de détection selon l'une quelconque des revendications 1 à 4, dans lequel le module de détection de pression (110) comprend en outre :

un premier élément de connexion (114), une extrémité du premier élément de connexion (114) étant connectée au profilé en porte-à-faux (111) et l'autre extrémité du premier élément de connexion (114) étant connectée à une carte principale du dispositif électronique, et le module de détection de pression (110) transmettant un signal électrique correspondant au signal de pression à la carte principale du dispositif électronique par l'intermédiaire du premier élément de connexion (114).

6. Dispositif de détection selon l'une quelconque des revendications 1 à 5, dans lequel

le module de détection de PPG (120) comprend en séquence un couvercle transmettant la lumière (121), une carte de circuit imprimé (122) et un second élément de connexion (123) depuis un côté faisant face au dispositif électronique de détection d'une pression de l'utilisateur vers le côté opposé ;

le couvercle transmettant la lumière (121) est configuré pour recevoir la pression de l'utilisateur ;

une source lumineuse (1221) et un détecteur de lumière (1222) sont soudés sur la carte de circuit imprimé (122), la source lumineuse (1221) est utilisée pour émettre un signal optique vers un doigt de l'utilisateur, et le détecteur de lumière (1222) est configuré pour recevoir un premier signal optique qui est émis par la source lumineuse (1221) et renvoyé par réflexion ou transmission du doigt et pour convertir le premier signal optique en signal de PPG ;

le second élément de connexion (123) est configuré pour transmettre le signal de PPG à la carte principale du dispositif électronique.

7. Dispositif de détection selon la revendication 6, dans lequel le module de détection de PPG (120) comprend en outre :

un premier support (124), un premier côté du premier support (124) faisant face au dispositif électronique de détection d'une pression de l'utilisateur étant connecté de manière fixe au couvercle transmettant la lumière (121), le premier support (124) étant pourvu à l'intérieur d'une première cavité, et au moins une partie du second élément de connexion (123) étant agencée dans la première cavité ; et
la carte de circuit imprimé (122) est agencée dans la première cavité, et la carte de circuit imprimé (122) et au moins une partie du second élément de connexion (123) sont connectées de manière fixe au premier support (124) par l'intermédiaire d'une couche de colle.

8. Dispositif de détection selon l'une quelconque des revendications 1 à 7, dans lequel l'espace entre le support coulissant (160) et le second support (170) est compris entre 30 um et 50 μm.

9. Dispositif de détection selon l'une quelconque des revendications 1 à 7, dans lequel un bloc d'arrêt est agencé au sommet d'un côté du second support (170) faisant face au dispositif électronique de détection d'une pression de l'utilisateur.

10. Dispositif de détection selon l'une quelconque des revendications 1 à 9, dans lequel une taille du second support (170) dans une première direction est supérieure à une taille du module de détection de PPG (120) dans la première direction, la première direction étant perpendiculaire à la seconde direction.

11. Dispositif de détection selon l'une quelconque des revendications 1 à 10, dans lequel le module de détection de pression (110) comprend en outre :

   un support de module (113), la base fixe (111b) étant fixée dans le dispositif électronique par l'intermédiaire du support de module (113) ;
   un premier élément d'étanchéité (180) adhérant à un cercle autour d'un côté du support de module (113) faisant face au dispositif électronique de détection d'une pression de l'utilisateur ; et
   un second élément d'étanchéité (190) agencé sur un côté du support de module (113) opposé au premier élément d'étanchéité (180).

12. Dispositif électronique, comprenant :

   une coque ;
   le dispositif de détection d'informations sur des composantes biologiques (100) selon l'une quelconque des revendications 1 à 11, le dispositif de détection d'informations sur des composantes biologiques étant au moins partiellement agencé dans la coque ; et
   une carte principale agencée dans la coque et connectée électriquement au dispositif de détection d'informations sur des composantes bio-

logiques (100).

FIG. 1

FIG. 2

FIG. 3

FIG. 4

FIG. 5

FIG. 6

FIG. 7

FIG. 8

FIG. 9

FIG. 10

FIG. 11

FIG. 12

100

FIG. 13

Electronic device 200

Shell 210

Biological characteristic
information detection
device 220

Main board 230

FIG. 14

**REFERENCES CITED IN THE DESCRIPTION**

**Patent documents cited in the description**

- GB 2578120 A
- US 2013296714 A1
- US 2014364749 A1